**Europäisches Patentamt**

**European Patent Office**

**Office européen des brevets**

(19)

(11) Publication number: **0 104 039**
**B1**

(12) **EUROPEAN PATENT SPECIFICATION**

(45) Date of publication of patent specification: **09.12.87**

(51) Int. Cl.⁴: **A 61 F 13/20**

(21) Application number: **83305319.2**

(22) Date of filing: **12.09.83**

(54) Tampons and applicators.

(30) Priority: **17.09.82 GB 8226622**
**11.02.83 GB 8303780**

(43) Date of publication of application:
**28.03.84 Bulletin 84/13**

(45) Publication of the grant of the patent:
**09.12.87 Bulletin 87/50**

(84) Designated Contracting States:
**AT BE FR GB SE**

(56) References cited:
**WO-A-82/02489**
**US-A-3 749 093**
**US-A-4 211 225**
**US-A-4 318 404**

(73) Proprietor: **Smith and Nephew Associated Companies p.l.c.**
**2, Temple Place Victoria Embankment**
**London WC2R 3BP (GB)**

(72) Inventor: **Lloyd, Ronald**
**63 Cambridge Road**
**Sawbridgeworth Hertfordshire (GB)**

(74) Representative: **Cole, William Gwyn**
**Corporate Patents Department Smith and Nephew Research Limited Gilston Park Harlow Essex CM20 2RQ (GB)**

Courier Press, Leamington Spa, England.

## Description

The present invention relates to tampons and applicators therefor, designed to aid insertion of the tampons into a bodily cavity and its extraction therefrom.

Conventional tampons can be relatively uncomfortable to insert into a bodily cavity such as a vagina due to friction between the walls of the bodily cavity and the surface of the tampon. United States Patent No. 4,211,225 discloses tampons shrouded by one or two water-permeable shrouds which ensure low friction between the outer shroud and the tampon. The shrouds are affixed to the nose of the tampon, which is inserted into the vagina in the shrouded conformation. On extraction, if the friction between vaginal wall and outer shroud is greater than that between either shroud and tampon, the shroud inverts, the rolling action of the inverting shroud making the extraction of the tampon relatively comfortable. The outer shroud may also be pre-creased circumferentially so as to facilitate the collapse and inversion of the shroud on withdrawal. Such a shroud, however, does not prevent friction between the shroud and the wall of the bodily cavity during insertion of the tampon. United States Patent Nos. 4,286,594 and 4,318,404 discloses an applicator which comprises a flexible sleeve which is inverted at one end and a tampon which is in contact with the inverted portion of the sleeve. In use the applicator is placed inside the vagina and pressure applied to the rear end of the tampon to expel the tampon from sleeve into the vagina. During insertion of the tampon the inverted portion of the sleeve everts and unrolls away from the surface of the tampon against the walls of the vagina to expose the moving surface of tampon to the vagina. Such an applicator therefore does not prevent friction between the wall of the bodily cavity and the surface of the tampon.

A tampon has now been found which substantially reduces contact between the wall of the bodily cavity and the surface of the tampon during the insertion of the tampon into the bodily cavity.

Accordingly, the present invention provides a tampon for insertion into a bodily cavity, which tampon comprises a flexible tube having an inverted collapsible portion extending from one end of the tube and a main tampon body which is capable of translational movement within the tube characterised in that the collapsible portion is attached to the main tampon body and part of said collapsible portion is in an axially compressed form at or about the nose of the main tampon body so that on insertion the axially compressed portion everts to form a sleeve about the moving tampon body.

On insertion of the tampon of the invention into the bodily cavity the inverted axially compressed collapsible portion of the flexible tube everts against the wall of the bodily cavity and forms a sleeve about the main tampon body which prevents contact between the bodily cavity and the moving surface of the tampon during insertion thereby reducing discomfort caused by frictional engagement of the tampons surface with the wall of the bodily cavity.

When used herein 'collapsible' means that the tube portion so described can collapse sufficiently radially to allow its inversion into the tube.

The tube of the present invention include circumferentially continuous tubes and tubes in the form of a rolled sheet.

Favourably, the main tampon body is a sliding fit in the tube, preferably a low-friction sliding fit. The tube may have an inner perimeter lesser, equal or greater than the maximum girth of the main tampon body, but if lesser it must be capable of ready circumferential expansion to accommodate the main tampon body.

The collapsible portion of the flexible tube is attached to or with the main tampon body. Preferably the collapsible portion is attached at or near a free end to the nose or tail of the main tampon body, or to near the nose or tail of the main tampon body. If the portion is attached in such a way that the everted portion shrouds the body in use, the portion must be of, or be capable of expanding to a sufficient diameter to accommodate the tampon body when expanded on absorption.

When used herein 'attached to' a given point means that that point is where articulation between the main tampon body and the collapsible portion occurs. For example, the tube may be attached to the conventional retrieval string near the body nose but constrained to or by the body so that articulation between body and tube is only possible at the body tail. The tube is then 'attached to' the tampon body tail.

In a particularly preferred embodiment the whole of the flexible tube is collapsible and is attached at or near one end to the nose or to near the nose of the main tampon body, and is attached at or near its other end to the tail or to near the tail of the main tampon body.

It will be apparent that in use as one end of the flexible tube everts the other will invert.

In all such embodiments the tampon body effectively blocks the bore of the flexible tube at the position of attachment. Thus, for example, the collapsible portion may be attached to the tail of the main tampon body. If, alternatively, the flexible portion receives and conforms to part of the tampon body after eversion, the portion must be capable of circumferential expansion to accommodate the body when expanded in use. For example, the flexible portion may be of continuous perimeter greater than the tampon body girth at the attachment position and axially pleated and attached at points on the pleating to the tampon body to allow for tampon body expansion. Alternatively, that part of the flexible portion which receives the tampon body after eversion may be provided with axial slits of sufficient length to allow accommodation of the expanded tampon body in use by circumferential distension of the slits.

If the tube is in the form of a rolled sheet, accommodation may be achieved by an effective mutual sliding of the turns of the roll in an unrolling action.

All the foregoing tampons may also be provided with a tampon guide to register with the bodily cavity.

Accordingly in another aspect the invention provides tampon assembly for the aiding the insertion of a tampon into a bodily cavity which assembly comprises a tampon of the invention and a tampon guide having an opening adapted to allow passage of the tampon and to be placed over the bodily cavity opening.

The tampon guide may be attached to the flexible tube, but if so it is preferably detachable therefrom, for example by means of a preset frangible connection broken for example by pressure continued after tube eversion, or by a water-soluble connection which is rapidly dissolved by bodily fluids in situ, for instance a polyvinyl alcohol adhesive.

In a further embodiment, a piston may be receivable within the flexible tube to form a tampon applicator.

Thus, in a further aspect, the invention provides an applicator for aiding the insertion of a tampon into a bodily cavity which applicator comprises a tampon of the invention and a piston capable of sweeping the tube so that the axially compressed collapsible portion everts under the action of the piston to form the sleeve about the moving tampon body.

In the applicator of the invention if the main tampon body is interposed between the piston and the collapsible portion, the action of the piston will be transmitted to the collapsible portion by the main tampon body. However, if the piston is tubiform and the main tampon body is within the piston and capable of translational motion within the piston then the piston will act directly on the flexible tube.

The applicator favourably has the collapsible portion, at or near its free end attached to the main tampon body, preferably at the nose or tail of the main tampon body or near the nose or tail of the main tampon body.

In a preferred embodiment, the applicator comprises a main tampon body, attached to the collapsible tube portion, and capable of translational motion within the flexible tube and a tubiform first piston, and a second piston capable of sweeping the first piston.

It will be seen that the first and second pistons correspond to the barrel and piston of a conventional vaginal tampon applicator, and that in this embodiment the first piston acts directly on the collapsible tube portion whilst the second piston either (more usually) acts on the main tampon body, or is tubiform like the first piston and also moves to house the tampon body and act on the collapsible tube portion.

Often the collapsible tube portion will be attached to with the main tampon body at or near the nose of the main tampon body, and the second piston will act on the main tampon body.

In this case the collapsible tube portion when inverted will evert under the action of the first piston to form a sleeve about the moving first piston and movement of the second piston to sweep the first piston and eject the main tampon body will cause further eversion of the collapsible tube portion to form a sleeve about the moving, ejected main tampon body.

This embodiment not only alleviates the relative discomfort of tampon insertion, but also the relative discomfort of tampon applicator insertion. This can be further beneficial, particularly for vaginal tampons, in minimising the risk of abrasion by the applicator and in allowing the use of conventional applicator barrels which are rougher and/or unstreamlined and thus potentially cheaper.

All the foregoing applicators may also comprise a piston guide.

Accordingly, in another aspect of the invention the applicator for aiding the insertion of a tampon into a bodily cavity, can comprise a piston guide receiving the piston and the tampon and having an opening to to be placed over the bodily cavity opening.

The piston guide may be attached to, but then preferably detachable from, the flexible tube, as described for tampon guides hereinbefore.

In the foregoing applicators, tampons and tampon assemblies of the invention the main tampon body will normally be provided with conventional retrieval means such as a string attached to the tampon body tail. Thus any piston will often be tubiform so that the retrieval means may be led through the piston. Such a retrieval means has the advantage that withdrawal is effected by inverting the collapsible portion into the tube. The rolling action of the inverting collapsible portion advantageously makes the extraction of the tampon relatively comfortable.

In all aspects of the present invention, it is preferred that at least a major proportion if not all the flexible tube is collapsible. The collapsible portion of flexible tube can be suitably 80% to 100% and preferably 100% of the total length of the flexible tube.

The walls of the collapsible portion of the flexible tube can suitably have an axially extending series of circumferential folds which alternate in direction, in for example a 'zig-zag' pattern. Such folds can be obtained by compressing a portion of the tube in a direction parallel to the axis of the tube. When a main tampon body is present, a favoured collapsible portion is thus capable of adopting a 'bellows' conformation for at least part of its length, preferably at the distal portion of the tube just before insertion.

When the collapsible portion is in its inverted conformation prior to use the collapsible portion of the 'bellows' type may be held in an axially compressed state. This can be achieved by adhesive means mechanical means or physical means. The adhesive means include the use of a water soluble adhesive for example a polyvinyl alcohol adhesive. Mechanical means can include an optionally water soluble wrapping film, Physical means include a heat set treatment.

When so compressed the axial 'bellows' length is favourably less than 15 mm and preferably 5—10 mm. When a main tampon body is present the compressed 'bellows' may be held on the tampon body nose by adhesive, mechanical or physical means as hereinbefore described.

The compressed 'bellows' conformation thus offers the advantage of compact storage of the tube collapsible portion before use.

Compact storage may also be achieved when a main tampon body is present by housing at least part of the collapsible portion, generally that part which is inverted, in an axial well extending within the tampon body from its nose.

The housed collapsible portion may be stuffed randomly into the well and/or be pre-folded for 'bellows' compression as hereinbefore defined. It may be retained in the well by for example mechanical means, such as an optionally water soluble wrapping film.

In use the everting inverted collapsible portion will pay out of the main tampon body nose, somewhat like a paying-out parachute.

The well may extend as far into the tampon body as is useful for convenient storage. For practicality it is preferred that it does not breach the body tail.

Such a well in a tampon for an adult vagina is favourably less than 8 mm, and preferably 3 to 6 mm in diameter.

Alternatively the well for holding the collapsible portion may be formed by placing a housing onto the nose of the tampon thereby defining a space between the nose of the tampon and the front wall of the housing. This space is capable of holding the collapsible portion which issues from a circular hole in the front wall of the housing.

Suitably the housing is formed from a plastics material, for example polypropylene, or from cardboard or water soluble or insoluble polymeric film or waxes which may be soluble, meltable and optionally contain a medicament.

Generally the housing will be fixed to the nose of the tampon by mechanical of adhesive means. Preferably the housing is a push fit onto the tampon. The end of the collapsible portion may be secured to the tampon by trapping between the housing and the tampon.

The internal diameter of the well may be up to 16 mm. This has the advantage that compared to the well hereinbefore described the housing will be shorter to hold a given length of track.

In a further aspect of the invention the nose portion of the main tampon body can have a diameter which is smaller than that of the remaining portion of the main tampon body to accommodate an axially compressed collapsible portion of the tube positioned about the nose of the main tampon body. Such a smaller diameter nose portion of main tampon body may be in the form of a front piece around which the track is held. The front piece may be adhered to the front of the tampon. Suitably an adhered front piece can be formed from similar material to that of the housing above. Preferably the smaller diameter nose portion is an integral part of the tampon body formed for example by compression during the production of the tampon body.

In all aspects of the present invention, either or both the internal and external surfaces of the flexible tube including the liner when present, especially those surfaces of the collapsible portion may be coated with a lubricant (which does not impair its water-permeability or solubility as apt) so that the tube everts easily under low pressure. Suitable lubricants include solid lubricants such as talc, liquid lubricants such as silicone oils and release coatings for example cured silicone resin release coatings, and greasy lubricants, such as glycerol esters of vegetable fatty acids, for example the 'Witepsols' (trade mark of Dynamit Nobel) and selected saturated unbranched vegetable fatty acid triglycerides such as the 'Dynasans' (trade mark of Dynamit Nobel AG).

Alternatively, the flexible tube may be made of a self-lubricating film, for example, of a self-lubricating plastic, such as a polyamide or polyurethane.

In all aspects of the present invention, the flexible tube is preferably inverted so that at least part of the collapsible portion of the flexible tube is inside the rest of the flexible tube. With this arrangement the collapsible portion will readily evert when pressure is applied to it. It will be appreciated that in some embodiments where all the flexible tube is collapsible the tube may be totally inverted so that no part of the tube actually lies within another.

The flexible tube used in this invention will be suitable for lining the walls of a bodily cavity. The size of the tampon will depend on the size of the cavity into which it is inserted. Suitable tampons will often be roughly circular in cross-section, optionally with conventional circumferential crimping on the main tampon body when present, or the flexible tube when a main tampon body is absent. Suitable tampons may have a maximum cross-dimension of 4 to 20 mm before use. Preferably, these tampons are vaginal tampons. Thus most suitably the tampons have a maximum girth similar to the average perimeter of a vaginal tract. A preferred tampon for an adult vagina has a maximum cross-dimension of 10 mm to 18 mm, before use. If the tampon comprises a shrouded tampon body or has a tube component surrounding an absorbent component, the flexible tube collapsible portion or tube component should preferably either be of, or be capable of expanding to, a diameter twice the above maximum cross-dimension to allow for expansion of the absorbent in use. If the collapsible portion is in the form of a roll, it is preferred that it is capable of still shrouding the main tampon body after expansion of the latter in use.

Similarly, when the tampon is inserted by digital or piston pressure on the flexible tube and/or a main tampon body when present the flexible tube must be capable of accommodating the digit or piston, or of expanding to do so.

The collapsible portion of the flexible tube of the invention when inserted into the vagina can be everted to form the lining for the walls of the vagina. The length of flexible tube used in this invention for the collapsible portion can suitably be 30 mm to 200 mm. The preferred length of flexible tube for the collapsible portion which is suitable for lining the walls of an adult vagina is from 40 mm to 175 mm depending of course on the particular embodiment. For example the type of tampon where the tube is attached to both ends of a main tampon body may be up to 120 mm longer than one attached to one end only.

The flexible tube will be made of a biocompatible material.

When the tampon comprises a main tampon body which is essentially completely shrouded by the sleeve formed by the fully everted collapsible tube portion, e.g. when the collapsible portion is attached at or near the nose of the main tampon body, the collapsible portion must be water-permeable or water-soluble at least in part so that the main tampon body can fulfil its normal absorbent function. Suitable collapsible tube structures include nets, non-woven, woven or knitted fabrics and porous, filmic materials. It is preferred that the collapsible portion of the flexible tube is made from a thin layer which is permeable to liquid water. Apt layers include flexible thin layers of nets and non-woven fabrics, such as spun-bondeds.

Suitable materials include plastics such as polyamides, polyolefins, for example polyethylene and polypropylene, and polyurethanes.

Suitable layers have a thickness of 25 μm to 400 μm and preferably a thickness of 75 μm to 150 μm, or of 10 to 160 g/m$^2$, preferably 30 to 60 g/m$^2$.

Favoured collapsible portions include spun-bonded polyamide non-woven, of about 13.5 g/m$^2$ (0.4 oz/yd$^2$), such as 'Cerex' Monsanto; spun-bonded polypropylene non-woven of about 20 g/m$^2$, such as 'Corovin SMED'; high density polyethylene net manufactured following UK 1 548 865, such as Net 909 Grade A4 (Smith and Nephew); and polyurethane net.

A preferred collapsible portion of the flexible tube comprises a layer of spun-bonded polypropylene of a thickness of 50 μm, in particular 'Corovin SMED'.

Alternatively, the collapsible portion of the flexible tube may be made at least in part from a filmic thin layer of a material which is itself impermeable to liquid water but perforate or microperforate to allow access of bodily exudates to the main tampon body. Suitable films have a thickness of 12.5 to 100 μm and preferably a thickness of 25 to 50 μm.

In all the above films the perforations are suitably of 1 to 55 mm$^2$ in area, distributed at $25 \times 10^4$ to $1 \times 10^4$ perforations per m$^2$, preferably 4 to 25 mm$^2$ in area distributed at $9 \times 10^4$ to $2 \times 10^4$ perforations per m$^2$.

Suitable materials include films of low density polyethylene, high density polyethylene, polypropylene, ethylene/vinylacetate copolymers, natural rubber, silicone elastomers and polyurethanes, including hydrophilic polyurethanes.

Preferred materials include high density polyethylene, and polyurethane, such as 'Estanes' (B. F. Goodrich & Co). Preferred Estanes are solution grade 5714 Fl and extrusion grade 58201.

Favoured collapsible portions include high-density polyethylene, of polyurethane, or 25 to 50 μm thickness having perforations of 4 to 25 mm$^2$ in area distributed at $9 \times 10^4$ to $2 \times 10^4$ perforations per m$^2$.

A preferred collapsible portion comprises a film of Estane 5714 Fl 20 to 30 μm thick and having perforations of 10 to 15 mm$^2$ in area distributed at $4 \times 10^4$ to $3 \times 10^4$ perforations/m$^2$.

As mentioned previously, the flexible tube collapsible portion may be water-permeable or water-soluble throughout, but it will suffice if all or part of that part of the everted collapsible portion which shrouds the tampon body is water-permeable or water-soluble; the remainder of the collapsible portion may be water-impermeable.

The collapsible portion may thus be a composite of a water-permeable or water-soluble part and a proximal water-impermeable part. The two parts may be attached by thermal bonding or an adhesive in peripherally extending mutual abutment or with axial overlap extending over part or all of the proximal water-impermeable part.

Suitable collapsible portion materials include those water-permeable materials recited hereinbefore, and those intrinsically water-impermeable materials recited hereinbefore. Suitable specific and film thicknesses within each class of materials are also those recited hereinbefore for each class.

When the tampon comprises a main tampon body which is essentially unshrouded by the sleeve formed by the fully everted collapsible tube portion, e.g. when the collapsible portion is attached at or near the tail of the main tampon body, the collapsible portion need not be water-permeable or water-soluble. Suitable collapsible tube structures include filmic materials which may preferably be water-impermeable. Suitable materials and film thicknesses are as recited hereinbefore for intrinsically water-impermeable collapsible tube portions.

The main tampon body may be of conventional absorbent materials such as the bulky non-wovens described hereinbefore.

An alternative when the tampon comprises a main tampon body essentially completely shrouded by the sleeve formed by the fully everted collapsible portion is that the collapsible portion is made of a water-soluble filmic material, so that once the tampon is in situ the collapsible portion of the tube dissolves.

In a preferred embodiment, the material dissolves to give a lubricative solution film, which does not clog the main tampon body but is sufficiently viscous not to be absorbed by the tampon body or to run out of the bodily cavity. The solution film then advantageously serves to render tampon extraction relatively comfortable.

In all embodiments especially for vaginal tampons where the flexible tube is water-permeable or water soluble, it is preferred that the proximal section of the collapsible portion and/or the (proximal) non-collapsible portion when present is made from a water-impermeable material to avoid 'wicking' of exudate to the bodily cavity opening. Suitable materials include polyurethanes, including hydrophilic polyurethanes, as films, or hydrophobic non-woven fabrics or such fabrics treated with silicones.

When present, a tampon guide must suitably be larger transversely than the bodily cavity into which the tampon is to be inserted.

The guide may be made of the same or similar materials as those recited hereinbefore for the absorbent component of a tampon according to the present invention which consists essentially of a flexible tube, that is bulky non-wovens and plastics foams, although since the prime function is not absorption other fabrics such as felts may be used.

The guide should of course not be deformable to the extent that it enters the bodily cavity during application of the tampon. This is especially the case where the guide is absorbent and more deformable after absorption of fluids. For this reason, less absorbent and/or less flexible materials than those used for tampon absorbent components may be preferred.

The guide may be integral with the tube. For example it may be formed as a rolled lip on the proximal edge of the tube held in position by gluing or thermal bonding. The guide may still be made separable from the tube as hereinbefore described.

Where the tampon guide is also a piston guide and either unattached to, or made to be detached from, the flexible tube, the guide may like a conventional tampon applicator barrel be made of the same material as the piston, such as plastics or cardboard optionally plastics coated.

The main tampon bodies used in the inventions which have the collapsible portion of the tube attached to or about the nose thereof can advantageously be provided with a shroud which surrounds a portion of the main tampon body which is to the rear of the nose of the tampon. Such a shroud prevents contact between the everting tube and the main tampon body and thus reduces friction there between when the tampon is inserted into the bodily cavity. Suitable shrouds will be normally made of a water permeable material coated with a lubricant material as described hereinbefore in relation to water permeable collapsible tubes. The shroud can be attached to the main tampon body for example at a circumferential line or area about the nose of the tampon by any convenient method which includes adhesive bonding, heat sealing or mechanical means. The shroud can conveniently be an extension of or attached to the collapsible portion of the tube in which case both of the components can be attached to main tampon body at the same circumferential line or area for example by means of a suitable band material such a cord.

A tampon in accordance with the present invention can be inserted into a bodily cavity by a method which comprises placing the tampon with the inverted collapsible portion of the tube proximal to the cavity, inserting the proximal part of the flexible tube into the cavity opening, and everting the collapsible portion of the tube within the cavity.

The flexible tube for use in the invention can be conveniently made by various methods depending on its structure and material.

Thus, for example when the collapsible portion is or comprises a peripherally continuous tube of a thin net, non-woven, woven or knitted fabric, the material in commercially available sheet form cut to suitable dimensions may be thermally bonded if thermoplastic, or glued with a biocompatible water-insoluble glue to form the tube. Knitted fabrics may be knitted in suitable tubular form.

Water-soluble, porous or intrinsically water-impermeable filmic materials may be thermally bonded or glued as above, as apt. Alternatively, filmic tubes may be formed by tubular extrusion, or by mandrel dipping in a suitable solution or latex or the tube material, followed by drying to form the film.

Intrinsically water impermeable sheet materials may be perforated or microperforated conventionally before or after fabrication into a tube. Other filmic materials may also be perforated or microperforated conventionally ater extrusion or mandrel dipping.

When the collapsible portions is or comprises a bulky non-woven this may be fabricated from a sheet into a tube by thermal bonding (if thermoplastic), gluing or stitching, or the non-woven may be produced in suitable tubular form. The tube is generally peripherally continuous.

Foams are suitably fabricated into a tube by mandrel dipping into a generated foam and drying on the mandrel to form the foam.

Suitable glues for tube fabrication include polyurethane, acrylic and cyanoacrylic adhesives.

When present, the main tampon body or piston will generally be made of materials conventional therefor in the art.

The collapsible portion of the flexible tube may be attached conventionally to the main tampon body by thermal bonding, if both portion and body are thermoplastic, by gluing with a biocompatible water-insoluble glue, or, when the collapsible portion is a fabric, by stitching-on. Alternatively if the main tampon body is provided with a conventional retrieval string, the tube is conveniently bonded, glued or even stitched to the string which is conventionally attached to the main tampon body.

Suitable glues include those mentioned hereinbefore.

Alternatively, the tube may be of the same material as the main tampon body, for example of viscose or cotton staple, when the tube may be fabricated as an integral attached part of the main tampon body.

Conventional tampon bodies are often formed by rolling a layer of absorbent, usually around a core retrieval string. A convenient roll-form tube attached to the main tampon body tail or nose may be fabricated by partially forming the body by rolling, overlaying the residual absorbent layer at least in part with a sheet of the tube material and continuing the rolling process to form the main tampon body and an interleaved roll-form tube.

The tampon may be provided with a topical anti-infective agent.

It is preferred that the devices of the invention are sterile to aid in preventing infection of the bodily cavity during insertion of the tampon. The device can be suitably packed into a bacteria-proof package and sterilised within the package by conventional methods.

Embodiments of the invention will now be described by way of example with reference to the accompanying drawings wherein:

Figs. 1 and 1a are longitudinal sections of a tampon assembly in accordance with the present invention;

Fig. 1b is a tampon of the present invention everted in situ after application from its tampon assembly.

Figs. 2 and 2a are longitudinal sections of a particularly preferred tampon in accordance with the present invention, Fig. 2b depicting the tampon fully everted in situ after application.

Figs. 3, 3a and 3b are longitudinal sections of a preferred applicator in accordance with the present invention, Fig. 3c depicting the corresponding tampon of the invention everted in situ after application.

Figs. 4 and 4a are longitudinal sections of a second particularly preferred tampon in accordance with the present invention, Fig. 4b depicting the corresponding tampon of the invention everted in situ after application.

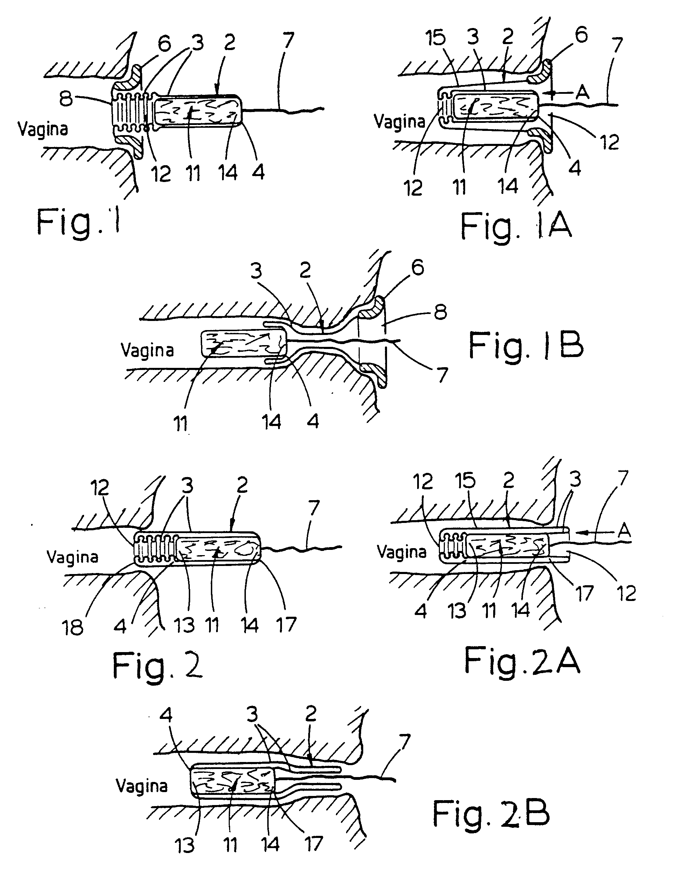

Figs. 1a to 4a depict in each case the relevant applicator, tampon assembly or tampon partially inserted into a vagina.

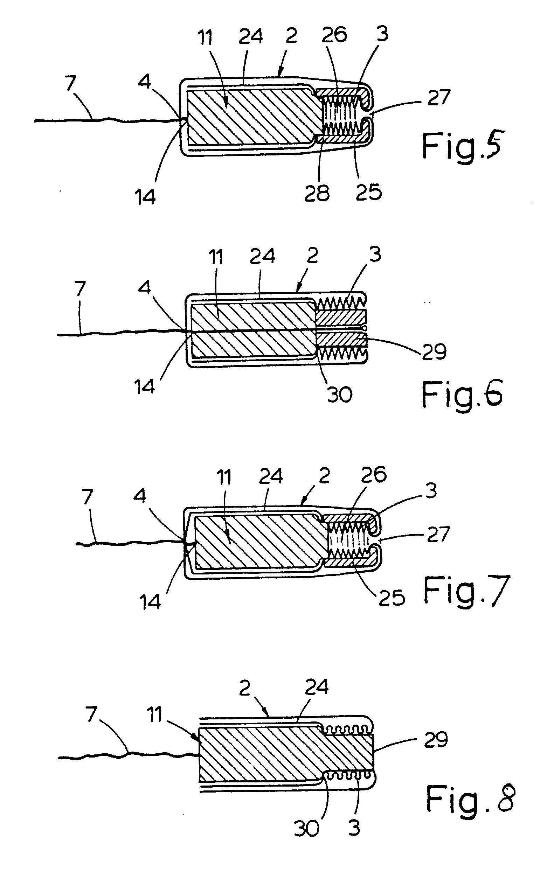

Fig. 5 shows a longitudinal cross-section through a further particularly preferred tampon in which the collapsible portion is held with a housing on the nose of the tampon.

Fig. 6 shows a longitudinal cross-section through an alternative embodiment in which the collapsible portion is held around a front piece of the tampon.

Fig. 7 shows an embodiment of a tampon shown in Fig. 5 in which the shroud and collapsible portion completely enclose the tampon and are attached to the withdrawal string.

Fig. 8 shows a longitudinal cross-section through a further preferred embodiment of the type of tampon shown in Fig. 6.

In Fig. 1 a tampon assembly in accordance with the present invention comprises a flexible tube (2) having an inverted collapsible portion (3) extending from one end (4) (in this embodiment, to the whole of the flexible tube (2)), and a tampon guide (6) receiving the flexible tube (2), and attached to the second end of the tube by a present frangible (or water-soluble) connection.

The end (4) of the collapsible portion (3) is attached to the tail (14) of the main tampon body (11) so that the tampon body (11) blocks the bore (12) of the tube (2). The tampon body is provided with a conventional string retrieval means (7).

The collapsible portion (3) is in part longitudinally compressed into a 'bellows' conformation in which it has been held for example with a wrapping film (removed in Fig. 1).

In Fig. 1 the tampon assembly is positioned with the opening (8) of the guide (6) just within the vaginal opening.

In Fig. 1a the inverted collapsible portion (3) of the tube (2) is partially everted under the action of a finger in a direction shown by arrow A; to form a sleeve (15) about the moving tampon body (11).

In Fig. 1b the collapsible portion (3) is fully everted to leave the main tampon body (11) essentially unshrouded. The collapsible portion (3) is thus preferably water-impermeable. After full eversion further digital pressure has been applied to break the frangible connection at end, and the tampon guide (6) has been removed.

In Fig. 2 a tampon in accordance with the present invention comprises a flexible tube (2) having a collapsible portion (3) extending from one end (4) (in this embodiment to the whole of the flexible tube (2)), and here inverted into the tube (2).

The end (17) of the collapsible portion (3) is attached to the tail (14) of a main tampon body (11) so that the tampon body (11) blocks the bore (12) of the tube (2), and the end (4) of the collapsible portion (3) is attached to the nose (13) of the main tampon body (11) so that the tampon body (11) thus blocks the bore (12) of the tube (2). The tampon body is provided with a conventional string retrieval means (7).

The collapsible portion (3) as in part longitudinally compressed into a "bellows' conformation in which it has been held for example with a wrapping film (removed in Fig. 2).

In Fig. 2, the tampon is positioned with its distal end (18) just within the vaginal opening.

In Fig. 2a, the inverted collapsible portion (3) of the tube (2) is partially everted under the action of a finger in a direction shown by arrow A, to form a sleeve (15) about the moving tampon body (11).

In Fig. 2b, the collapsible portion (3) is fully everted to keep the main tampon body (11) essentially shrouded. The collapsible portion (3) is thus preferably water-permeable.

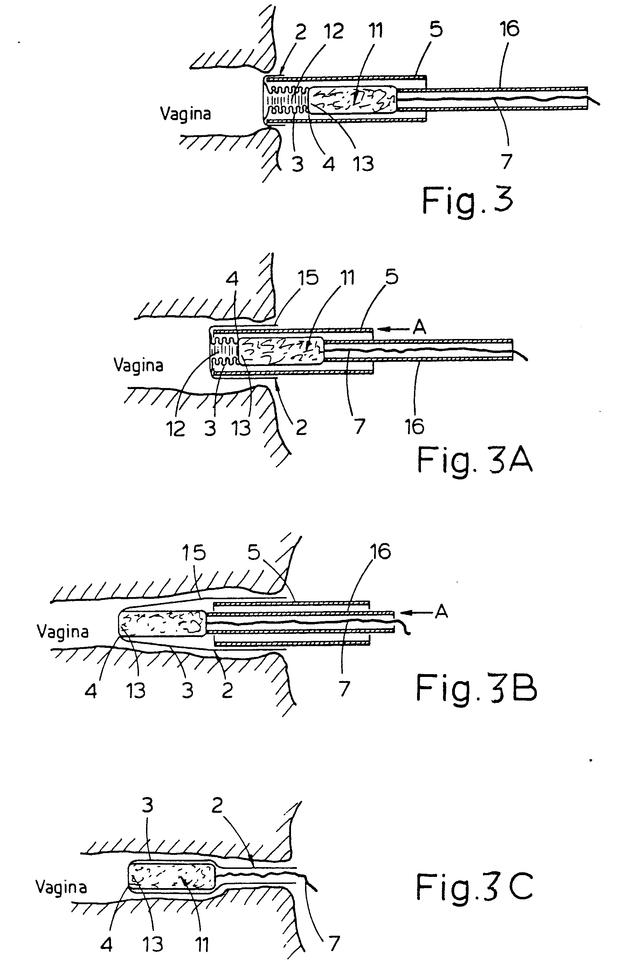

In Fig. 3 an applicator in accordance with the present invention comprises a flexible tube (2) having a collapsible portion (3) extending from one end (4) (in this embodiment, to be the whole of the flexible tube (2)), and here inverted into the tube (2), a conventional main tampon body (11) capable of translational movement within the tube (2) and lying within a tubiform first piston (5) which is capable of sweeping the tube (2) to evert the inverted collapsible portion (3), and a second piston (16) capable of sweeping the first piston (5) to eject the main tampon body (11) therefrom.

The end (4) of the collapsible portion (3) is attached to the main tampon body (11), so that the tampon body (11) blocks the bore (12) of the flexible tube (2). The collapsible portion (3) is attached at its free end (4) to near the nose (13) of the tampon body (11). The main tampon body is provided with a conventional string retrieval means (7) led through the tubiform piston (16).

The collapsible portion (3) is in part longitudinally compressed into a 'bellows' conformation in which it has been held for example with a wrapping film (removed in Fig. 3).

In Fig. 3 the tampon assembly is positioned with the first piston (5) just within the vaginal opening.

In Fig. 3a the inverted collapsible portion (3) is partially everted under the action of the first piston (5) acting directly on the collapsible portion (3), in a direction shown by arrow A, to form a sleeve (15) about the moving first piston (5), by pulling the collapsible portion (3) off the front of its bellows conformation.

In Fig. 3b the inverted collapsible portion (3) of the tube (2) is further everted under the action of the second piston (16) on main tampon body (11) in a direction shown by arrow A; to form a sleeve (15) about the moving tampon body (11), by pushing the 'bellows' part of the collapsible portion (3) out of the first piston (5).

In Fig. 3c the collapsible portion (3) is fully everted to leave the main tampon body (11) shrouded. The collapsible portion (3) is thus water-permeable. After full eversion both pistons (5) and (16) have been removed.

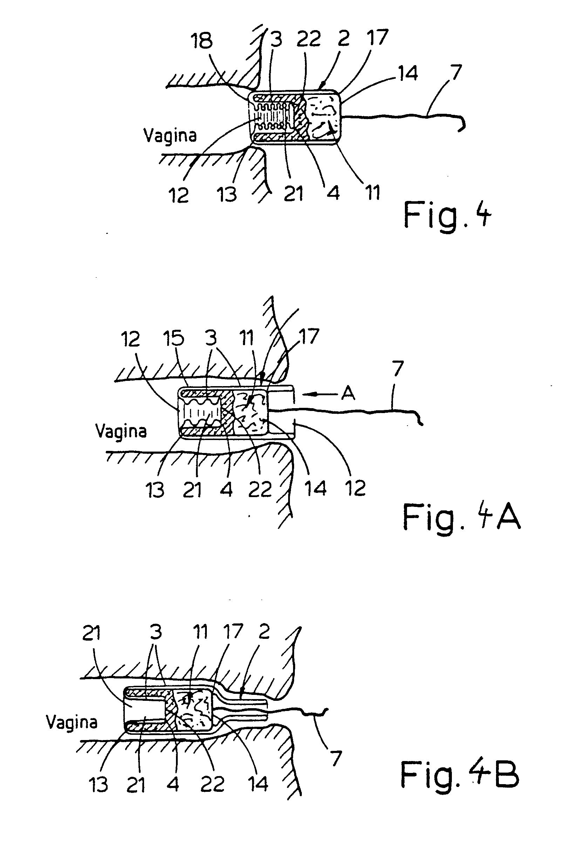

In Fig. 4, a tampon in accordance with the present invention comprises a flexible tube (2) having a collapsible portion (3) extending from one end (4) to be the whole of the flexible tube (2) and here inverted into the tube (2).

The end (17) of the collapsible portion (3) is attached to the tail (14) of a main tampon body (11) so that the tail (14) of the tampon body (11) blocks the bore (12) of the tube (2). The main tampon body (11) is provided with an axial well (21) extending from its nose (13) and having a floor (22). The second end (4) of the collapsible portion (3) of the tube (2) is attached to the floor (22) of the well (21) so that the floor (22) within the tampon body (11) also blocks the bore (12) of the tube (2).

In this embodiment, the inverted collapsible tube portion (3) is longitudinally compressed within the well (21) into a 'bellows' conformation. It has been held in this state in the well (21) for example with a wrapping film (removed in Fig. 4). Alternatively the inverted collapsible tube portion (3) may be randomly stuffed into the well (21).

Although the end (4) of the collapsible tube portion (3) is depicted as attached to the floor (22) of the well (21) in the tampon body (11), this end (4) may alternatively be attached to the nose (13) of the tampon body (11) and the inverted collapsible tube portion (3) housed within the well (21) as hereinbefore described.

In Fig. 4, the tampon is positioned with its distal end (18) just within the vagina opening.

In Fig. 4a, the inverted collapsible portion (3) of the tube has been partially everted and paid out of the well (21) under the action of a finger in the direction shown by arrow A to form a sleeve (15) about the moving tampon body (11).

In Fig. 4b, the collapsible portion (3) is fully everted to essentially shroud all of the tampon body (11) except well floor (22). The collapsible portion is thus preferably water-permeable.

If alternatively the end (4) of the collapsible tube portion (3) is attached to the nose (13) of the tampon body (11) the walls and floor (22) of the well (21) are unshrouded by the tube portion (3) which need not necessarily be water-permeable.

Figure 5 shows a particularly preferred embodiment of the present invention in which the collapsible portion (3) is retained between the nose of the tampon (11) and the front wall of a housing (25) which is held by a push fit onto the nose of the tampon. The space (26) present between the nose of the tampon (11) and the front wall of the housing (25) is capable of holding the collapsible portion (3) which is able to issue from a hole (27) in the front wall of the housing as the tampon is inserted into the vagina. In this embodiment the non-compressed portion of the tube (2) encloses the tampon and is fastened to the withdrawal cord (7) at the point (4). There is also present a shroud (24) which lies around the tampon inside the track and is fastened at point (28) by being trapped between the housing and the tampon. The end of the compressed portion of the tube is also fixed at this point. The presence of the shroud (24) reduces the friction between the tampon and the tube as the tampon is inserted, thus the sliding contact down the side of the tampon is non-woven against non-woven. Typically the non-woven is a Cerex material.

Figure 6 shows an alternative embodiment of the type of tampon shown in Figure 5 in which the housing (25) is replaced by a front piece (29) which is present on the nose of the tampon. The front piece may be formed from the same material as the tampon or it may be a non-absorbent material. In this embodiment the collapsible portion (3) is held around the front piece, its end being fixed to the tampon at point (30). The end of the internal shroud (24) is also conveniently fixed at this point. The withdrawal string (7) runs the length of the tampon from the end of the front piece exiting at the tail of the tampon (14).

Figure 7 shows a variation of the tampon described in Figure 5 in which the shroud also totally encloses the absorbent part of the tampon and is fastened to the withdrawal cord at point (4).

Figure 8 shows a further preferred embodiment of the type of tampon shown in Figure 6 in which the front piece (29) on the nose of the tampon (11) forms part of the main tampon body for example a compressed portion of the tampon. In this embodiment the collapsible portion (3) of the flexible tube (2) in a compressed 'bellows' configuration is positioned on the front piece (29) and attached around its circumference at (30). The collapsed 'bellows' portion of the tube (2) had been treated during its formation for example by a heat set treatment to maintain it in compressed state. The uncollapsed portion of the tube (2) is shown in a position surrounding the tampon (11) and shroud (24). The shroud (24) is conventionally an extension of flexible tube (2) and therefore also attached to tampon (11) at position (30). The withdrawal string (7) is attached to the tail of the tampon.

The pressure applied to the collapsible portion will of course be an axial pressure. The pressure applied to the collapsible portion may be applied directly (for example by using a piston as hereinbefore described) or may be applied indirectly by pressing the main tampon body. In one preferred form of the invention the pressure is applied digitally to one end of the main tampon body.

In preferred forms of this invention the collapsible portion is attached at or about the nose of the main tampon body.

## Claims

1. A tampon for insertion into a bodily cavity, which tampon comprises a flexible tube (2) having an inverted collapsible portion (3) extending from one end (4) of the tube and a main tampon body (11) which is capable of translational movement within the tube characterised in that the collapsible portion (3) is attached to the main tampon body (11) and part of said collapsible portion (3) is in an axially compressed form at or about the nose (13) of the main tampon body so that on insertion the axially compressed collapsible portion everts to form a sleeve (15) about the moving tampon body.

2. A tampon as claimed in claim 1 in which the collapsible portion (3) of the tube (2) is attached at an end (4) thereof to or about the nose (13) of the main tampon body (11).

3. A tampon as claimed in claim 2 which is a digital tampon in which the whole of the flexible tube (2) is collapsible and is attached at its other end (17) to the tail or near to the tail (14) of the main tampon body (11).

4. A tampon as claimed in any of claims 1 to 3 in which the nose portion (29) of the main tampon body (11) has a diameter smaller than that of the remaining portion of the main tampon body to accommodate an axially compressed collapsible portion of the tube positioned about the nose of the main tampon body.

5. A tampon as claimed in any of claims 2 to 4 in which the main tampon body (11) has a shroud (24) which surrounds a portion thereof which is to the rear of the nose of the tampon to reduce friction between the everting axially compressed collapsible portion of the tube and the main tampon body when the tampon is inserted into the bodily cavity.

6. A tampon as claimed in any of claims 1 to 5 in which the collapsible portion (3) of the tube comprises a water permeable non-woven fabric.

7. A tampon assembly for aiding the insertion of a tampon into a bodily cavity which assembly comprises a tampon according to claim 1 and a tampon guide (6) having an opening (8) adapted to allow passage of the tampon and to be placed over the bodily cavity opening.

8. An applicator for aiding the insertion of a tampon into a bodily cavity which applicator comprises a tampon according to claim 1 and a piston (5) capable of sweeping the tube (2) so that the axially compressed collapsible portion everts under the action of the piston to form the sleeve (15) about the moving tampon body.

9. An applicator as claimed in claim 8 in which the piston (5) is tubiform and the main tampon body (11) is within the piston and capable of translational motion therein so that the piston acts directly on the flexible tube (2).

10. An applicator as claimed in either of claims 8 or 9 which comprises a piston guide (6) receiving the piston and the tampon having an opening (8) to be placed over the body cavity opening.

## Patentansprüche

1. Tampon für die Einführung in einen Körperhohlraum, wobei der Tampon ein flexibles Rohr (2) mit einem sich von dessen einem Ende (4) aus erstreckenden, umgestülpten, zusammenlegbaren Abschnitt (3) und einen in dem Rohr verschiebbaren Haupttamponkörper (11) umfaßt, dadurch gekennzeichnet, daß der zusammenlegbare Abschnitt (3) an dem Haupttamponkörper (11) befestigt ist und ein Teil des zusammenlegbaren Abschnittes (3) an der oder um die Nase (13) des Haupttamponkörpers herum in axial zusammengepreßter Form vorliegt, so daß sich der axial zusammengepreßte, zusammenlegbare Abschnitt beim Einführen unter Bildung einer Hülse (15) um den sich bewegenden Tamponkörper herum nach außen kehrt.

2. Tampon nach Anspruch 1, bei dem der zusammenlegbare Abschnitt (3) des Rohres (2) an dessen einem Ende (4) an der oder um die Nase (13) des Haupttamponkörpers (11) herum angebracht ist.

3. Tampon nach Anspruch 2 in Fingerform, bei dem das ganze flexible Rohr (2) zusammenlegbar und an seinem anderen Ende (17) am Endstück oder nahe dem Endstück (14) des Haupttamponkörpers (11) angebracht ist.

4. Tampon nach irgendeinem der Ansprüche 1 bis 3, bei dem der Nasenabschnitt (29) des Haupttamponkörpers (11) einen Durchmesser aufweist, der kleiner ist als der des übrigen Abschnittes des Haupttamponkörpers, um eine Anpassung an einen axial zusammengepreßten, zusammenlegbaren Abschnitt des Rohres zu erreichen, der um die Nase des Haupttamponkörpers herum angeordnet ist.

5. Tampon nach irgendeinem der Ansprüche 2 bis 4, bei dem der Haupttamponkörper (11) eine Umhüllung (24) aufweist, die einen Abschnitt davon umgibt, der hinter der Nase des Tampons liegt, um die Reibung zwischen dem sich nach außen kehrenden axial zusammengepreßten, zusammenlegbaren Abschnitt des Rohres und dem Haupttamponkörper zu verringern, wenn der Tampon in den Körperhohlraum eingeführt wird.

6. Tampon nach irgendeinem der Ansprüche 1 bis 5, bei dem der zusammenlegbare Abschnitt (3) des Rohres ein wasserdurchlässiges Vlies umfaßt.

7. Tamponsatz zur Unterstützung der Einführung eines Tampons in einen Körperhohlraum, der einen Tampon nach Anspruch 1 und eine Tamponführung (6) mit einer Öffnung (8) umfaßt, die den Durchgang des Tampons und die Anordnung über der Öffnung des Körperhohlraums gestattet.

8. Applikator zur Unterstützung der Einführung eines Tampons in einen Körperhohlraum, der einen Tampon nach Anspruch 1 und einen Kolben (5) umfaßt, der über das Rohr (2) zu gleiten imstande ist, so daß sich der axial zusammengepreßte, zusammenlegbare Abschnitt unter der Wirkung des Kolbens unter Bildung der Hülse (15) um dan sich bewegenden Tamponkörper herum nach außen kehrt.

9. Applikator nach Anspruch 8, bei dem der Kolben (5) Rohrform hat und der Haupttamponkörper (11) sich verschiebbar innerhalb des Kolbens befindet, so daß der Kolben direkt auf das flexible Rohr (2) wirkt.

10. Applikator nach Anspruch 8 oder 9 mit einer Kolbenführung (6) zur Aufnahme von Kolben und Tampon mit einer über einer Öffnung des Körperhohlraums anzuordnenden Öffnung (8).

**Revendications**

1. Tampon à introduire dans une cavité corporelle, lequel tampon comprend un tube souple (2) ayant une portion (3) rabattable retournée se prolongeant depuis une extrémité (4) du tube et un corps principal du tampon (11) qui peut subir un mouvement de translation à l'intérieur du tube, caractérisé en ce que la portion rabattable (3) est fixée au corps principal du tampon (11) et une partie de cette portion rabattable (3) est sous une forme comprimée axialement sur le nez (13) ou autour du nez du corps principal du tampon, si bien que lors de l'introduction, la portion rabattable comprimée axialement se retourne en formant une gaine (15) autour du corps de tampon qui se déplace.

2. Tampon suivant la revendication 1, dans lequel la portion rabattable (3) du tube (2) est fixée à une extrémité (4) de celui-ci ou autour du nez (13) du corps principal du tampon (11).

3. Tampon suivant la revendication 2, lequel est un tampon digital dans lequel la totalité du tube souple (2) est rabattable et ce tube est fixé à son autre extrémité (17) à la queue (14) du corps principal du tampon (11) ou au voisinage de celle-ci.

4. Tampon suivant l'une quelconque des revendications 1 à 3, dans lequel la portion du nez (29) du corps principal du tampon (11) a un diamètre inférieur à celui de la portion restante du corps principal du tampon pour loger une portion rabattable comprimée axialement du tube disposée autour du nez du corps principal de tampon.

5. Tampon suivant l'une quelconque des revendications 2 à 4, dans lequel le corps principal du tampon (11) a une enveloppe (24) qui entoure une portion de celui-ci située à l'arrière du nez du tampon pour réduire le frottement entre la portion du tube rabattable comprimée axialement et se retournant et le corps principal du tampon lorsque le tampon est introduit dans la cavité corporelle.

6. Tampon suivant l'une quelconque des revendications 1 à 5, dans lequel la portion rabattable (3) du tube comprend un tissu non-tissé perméable à l'eau.

7. Assemblage de tampon contribuant à l'introduction d'un tampon dans une cavité corporelle, lequel assemblage comprend un tampon suivant la revendication 1, et un guide de tampon (6) ayant une ouverture (8) adaptée pour permettre le passage du tampon et devant être placée sur l'orifice de la cavité corporelle.

8. Applicateur contribuant à l'introduction d'un tampon dans une cavité corporelle, lequel applicateur comprend un tampon suivant la revendication 1 et un piston (5) capable de balayer le tube (2), si bien que la portion rabattable comprimée axialement se renverse sous l'action du piston pour former une gaine (15) autour du corps du tampon qui se déplacé.

9. Applicateur suivant la revendication 8, dans lequel le piston (5) est tubiforme et le corps principal du tampon (11) est à l'intérieur du piston et peut subir un mouvement de translation dans celui-ci, si bein que le piston agit directement sur le tube souple (2).

10. Applicateur suivant la revendication 8 ou la revendication 9, qui comprend un guide de piston (6) recevant le piston et le tampon, ayant une ouverture (8) à placer sur l'orifice de la cavité corporelle.

Fig. 1

Fig. 1A

Fig. 1B

Fig. 2

Fig. 2A

Fig. 2B

0 104 039

Fig. 3

Fig. 3A

Fig. 3B

Fig. 3C

2

Fig. 4

Fig. 4A

Fig. 4B

3

Fig.5

Fig.6

Fig.7

Fig.8